# EUROPEAN PATENT SPECIFICATION

(11) **EP 1 500 367 B1**
(45) Date of publication and mention of the grant of the patent: **05.03.2008**
(21) Application number: 03795422.9
(22) Date of filing: 12.09.2003
(51) Int. Cl.: A61B 1/04

(54) **IMAGE PROCESSING DEVICE AND IMAGE PICKUP DEVICE**
BILDBEARBEITUNGSVORRICHTUNG UND BILDAUFNAHMEVORRICHTUNG
DISPOSITIF DE TRAITEMENT D'IMAGES ET DISPOSITIF D'ANALYSE D'IMAGES

(30) Priority: 13.09.2002 JP 2002268834; 23.07.2003 JP 2003200814
(43) Date of publication of application: 26.01.2005
(73) Proprietor: Olympus Corporation, Tokyo 151-0072 (JP)
(72) Inventor: SUDO, Masaru, Olympus Med. Systems Corp.,, Shibuya-ku, Tokyo 151-0072 (JP)
(74) Representative: von Hellfeld, Axel
(86) International application number: PCT/JP2003/011695
(87) International publication number: WO 2004/023988

(56) References cited:
- JP-A- 2000 287 203
- JP-A- 2001 197 487
- US-B1- 6 339 446

## Description

### Technical Field

The present invention relates to an image processing apparatus and an image pick-up apparatus. More particularly, the present invention relates to an image processing apparatus and an image pick-up apparatus which can pick up images on monitors on different scanning systems for display and which further can record a still image by a high-quality video signal on a progressive system.

### Background Art

Conventionally, the promotion of miniaturization and pixel increase for a solid image pick-up device (hereinafter, referred to as a CCD (Charge Coupled Device)) results in the development and practical use for an electronic endoscope apparatus which picks up an image of an affected part of the body as a target of the diagnosis and cure by using the above-mentioned CCD.

The electronic endoscope apparatus photoelectrically converts a subject image serving as an image of the affected part as the target of the diagnosis and cure, which is projected on an image pick-up plane of the CCD, generates an image pick-up signal, converts the signal into a video signal on a general TV system, e.g., an NTSC or PAL system, and displays the video signal on a monitor as a video image or records it on a recording medium.

The TV uses an interlace video signal on the interlace scanning by which horizontal scanning lines are interlace-scanned every odd line and even line.

As compared with the interlace video signal (hereinafter, referred to as the interlace video signal), attention is recently paid to a progressive scanning as a non-interlace sequential scanning, by which the vertical resolution of video images displayed on the monitor is improved and the flicker of scanning lines is solved.

A video signal generated on the progressive scanning is inputted to a monitor which can display a progressive video signal (hereinafter, referred to as the progressive video signal) and a recording device which can record the progressive video signal and, thus the video image with high quality can be displayed and be recorded.

On the other hand, it is required that the monitor and recording device corresponding to the conventional interlace video signal can display and record the progressive video signal.

Japanese Unexamined Patent Application Publication No. 2001-197487 suggests an electronic endoscope apparatus in response to the above-described requirement, which converts a non-interlace progressive image pick-up signal generated by driving the CCD into a progressive video signal suitable to a progressive monitor or recording device, then, outputs the converted signal thereon, and converts the progressive video signal into an interlace video signal of a still image, and then outputs the converted signal on the monitor or recording device upon receiving an instruction for displaying the still image.

Further, Japanese Unexamined Patent Application Publication No. 2000-287203 suggests an electronic endoscope apparatus which generates a video signal based on an image pick-up signal from the CCD which is driven by any of the progressive scanning signal or interlace one.

Then, the picked-up subject image is often recorded as the still image upon observation using the endoscope. A method for recording the still image includes two methods of a field freeze for suppressing the image blur to the subject motion and a frame freeze for capturing the image with high vertical resolution. However, the above freezes have drawbacks that the vertical resolution is reduced in the field freeze and the subject motion is displayed as the image blur by the time difference of fields in the frame freeze.

On the contrary, the still image comprising the progressive video signal highly maintains the vertical resolution and can obtain the still image having high quality without the image blur due to the subject motion.

However, the electronic endoscope apparatus suggested in Japanese Unexamined Patent Application Publication No. 2000-287203 can switch the CCD to progressive signal processing or interlace signal processing, stores the video signal through the progressive signal processing or interlace signal processing on an image memory and generates a video signal for regenerating and display on the progressive signal processing or interlace one from the video signal thereon which is stored in the image memory. Therefore, if the video signal stored in the image memory comprises the still image comprising the interlace video signal, it becomes the interlace video signal.

The electronic endoscope apparatus suggested in Japanese Unexamined Patent Application Publication No. 2001-197487 converts, into the interlace video signal, the progressive video signal picked up and generated on the progressive system, outputs the converted signal, and records the still image comprising the interlace video signal on an external recording device.

That is, the electronic endoscope apparatuses disclosed in the above mentioned publication record the interlace video signals of the still image and therefore cause the above-mentioned image blur upon regenerating the interlace image signal of the recorded still image.

In consideration of the foregoing circumstances, it is an object of the present invention to provide an image processing apparatus and an image pick-up apparatus, which can display the progressive video signal picked up and generated by image pick-up means that can pick up an image comprising progressive signals with high quality on monitors having different scanning systems for display and which can record the progressive video signal upon recording the still image.

### Disclosure of Invention

According to the present invention, an image processing apparatus according to claim 1 and an image pickup apparatus according to claim 9 are provided.

### Brief Description of the Drawings

Fig. 1 is a block diagram showing the structure of an electronic endoscope apparatus according to a first embodiment of the present invention.
Fig. 2 is an explanatory diagram describing a regenerated image from the electronic endoscope apparatus according to the first embodiment of the present invention.
Fig. 3 is a block diagram showing the entire structure of an electronic endoscope system according to the first embodiment of the present invention.
Fig. 4 is a block diagram showing the structure of an electronic endoscope apparatus according to a modification of the first embodiment of the present invention.
Fig. 5 is a block diagram showing the structure of an electronic endoscope apparatus according to a second embodiment of the present invention.
Fig. 6 is an explanatory diagram describing a display screen of a subject still-image which is picked up by the electronic endoscope apparatus according to the second embodiment of the present invention.
Fig. 7 is an explanatory diagram describing a display screen of a plurality of still images picked up by the electronic endoscope apparatus according to the second embodiment of the present invention.
Fig. 8 is a block diagram showing the structure of an electronic endoscope apparatus according to a modification of the second embodiment of the present invention.

### Best Mode for Carrying Out the Invention

Hereinbelow, embodiments of the present invention will be described in detail with reference to the drawings.

### (First embodiment)

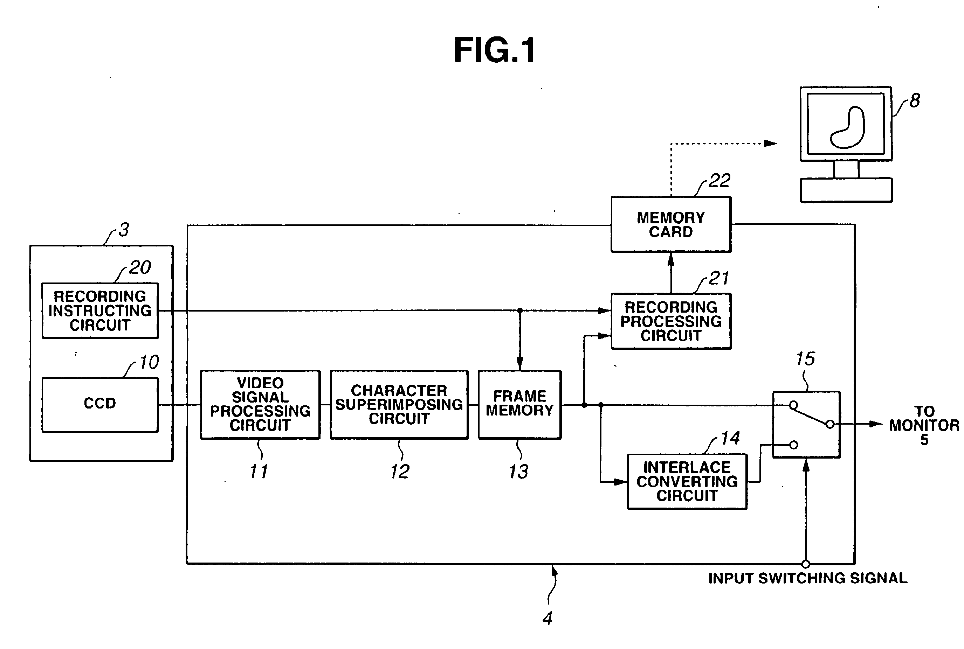

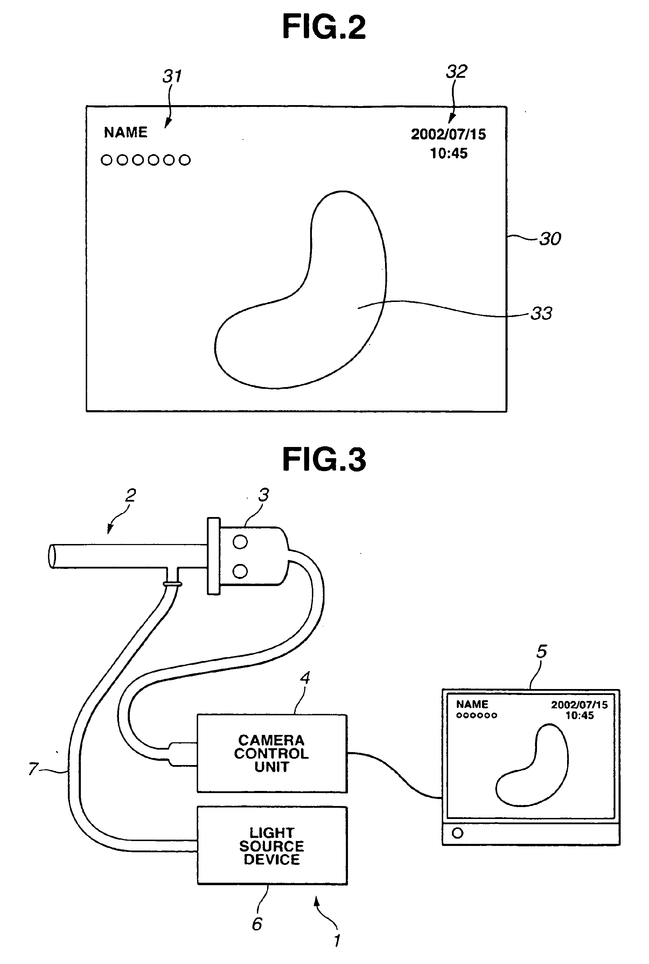

Fig. 1 is a block diagram showing the structure of an electronic endoscope apparatus according to the first embodiment of the present invention, Fig. 2 is an explanatory diagram describing a regenerated image from the electronic endoscope apparatus according to the first embodiment of the present invention, and Fig. 3 is a block diagram showing the entire structure of an electronic endoscope system according to the first embodiment of the present invention.

First, a description is given of the structure of an electronic endoscope system 1 according to the first embodiment of the present invention with reference to Fig. 3. The electronic endoscope system 1 comprises: an optical endoscope 2 which is inserted in the body cavity and which captures a subject image of an observed portion in the body cavity; a camera head 3 which is detachably connected to an eye piece of the optical endoscope 2; a camera control unit (hereinafter, referred to as a CCU (Camera Control Unit)) 4 serving as an image processing apparatus which drives a CCD serving as an image pick-up apparatus arranged in the camera head 3, which performs predetermined signal processing of an image pick-up signal photoelectrically converted and generated by the CCD, and which generates a standard video signal; a monitor 5 which displays the subject image based on the video signal outputted from the CCU 4; a light source device 6 having a light source which emits illuminating light; and a light guide cable 7 guiding the illumination light from the light source device 6 which is connected to the optical endoscope 2.

A description is given of the structure of the camera head 3 and the CCU 4 in the electronic endoscope system 1 with reference to Fig. 1. The image pick-up apparatus comprises the camera head 3 and the CCU 4.

The camera head 3 comprises: a CCD 10 which picks up the subject image transmitted from the optical endoscope 2; and a recording instructing switch 20 which instructs the recording of the subject image picked up by the CCD 10 as a still image. The CCD 10 generates and outputs pixel data of a progressive image pick-up signal serving as the non-interlace sequential scanning one, based on a driving signal inputted from CCD driving control means (not shown).

The CCU 4 comprises: a video signal processing circuit 11; a character superimposing circuit 12; a frame memory 13; an interlace converting circuit 14; a video signal output selector (hereinafter, referred to as a P/I selector (progressive/interlace selector)) 15; and a recording processing circuit 21. The video signal processing circuit 11 is video signal processing means which captures the pixel data of the progressive image pick-up signal picked up and generated by the CCD 10, and which performs signal processing for converting the pixel data of the progressive image pick-up signal into the progressive video signal. The character superimposing circuit 12 is a circuit which superimposes various character information to the progressive video signal processed and generated by the video signal processing circuit 11. The frame memory 13 is a memory which temporarily stores one frame of the progressive video signal having the character information superimposed by the character superimposing circuit 12, and which outputs the stored one frame of the progressive video signal. The interlace converting circuit 14 is converting means which converts in formats and outputs the progressive video signal outputted from the frame memory 13 into the interlace video signal. The P/I selector 15 is a switch device which can selectively output any of the progressive video signal outputted from the frame memory 13 and the interlace signal converted by the interlace converting circuit 14. The recording processing circuit 21 is a recording device which reads the stored frame of the progressive video signal in the frame memory 13 upon receiving an instruction for recording from the recording instructing switch 20 in the camera head 3, and which records the read frame of the progressive video signal to the memory card 22.

The video signal processing circuit 11 converts the progressive image pick-up signal outputted from the CCD 10 into a digital signal by using a correlation double sampling circuit, an A/D converter, a digital signal processor, etc., based on the driving signal inputted from the CCD driving control means (not shown) in the CCD 10, and performs processing including the interpolation, contour correction, white balance, and y correction of a color signal, luminance signal, pixel data so as to generate the progressive video signal.

The character superimposing circuit 12 is means which superimposes the character information indicating the diagnosis and cure date and the patient name onto the progressive video signal.

The frame memory 13 is a memory which stores, if necessary, the progressive video signal per frame unit having the character information superimposed by the character superimposing circuit 12 onto the progressive video signal, and which outputs the stored progressive video signal per frame unit.

The interlace converting circuit 14 is a circuit which converts in the format, the progressive video signal into the interlace video signal.

The P/I selector 15 as the switch means is a switch which selectively outputs any of the progressive video signal outputted from the frame memory 13 and the interlace video signal converted in the format by the interlace converting circuit 14. The video signal selected by the P/I selector 15 is outputted to the monitor 5 for displaying the video signal, via an output terminal (not shown).

That is, as the monitor 5 connected to the output terminal of the P/I selector 15, a monitor being able to regenerate the progressive video signal or a monitor being able to regenerate the interlace video signal is selected. Further, the P/I selector 15 selectively outputs, to the output terminal, the video signal corresponding to the scanning system of the monitor connected to the P/I selector 15.

The format of the video signal outputted from the P/I selector 15 is selected, that is, is set in accordance with a switch signal outputted by operating a monitor switch arranged to a front panel (not shown) of the CCU 4 by an operator according to the system of the connected monitor 5. Alternatively, the format of the video signal outputted from the P/I selector 15 is selected based on a switch signal according to the system of the connected monitor 5 which is automatically detected by automatic detecting means (not shown).

Namely, the progressive image pick-up signal is generated by reading data on the entire pixels which is picked up by the sequential scanning by the CCD 10 in the camera head 3 at the speed of 1/60 sec. The video signal processing circuit 11 processes the progressive image pick-up signal, thus to generate the progressive video signal. The character superimposing circuit 12 superimposes the character information on the progressive video signal, and the progressive video signal having the superimposed character information is temporarily stored in the frame memory 13 every frame.

In other words, the frame memory 13 outputs the progressive video signal (moving image progressive signal) as a moving image signal obtained by updating the stored frame every 1/60 sec. The progressive video signal outputted from the frame memory 13 is inputted to the interlace converting circuit 14 and the P/I selector 15.

The interlace converting circuit 14 divides the non-interlace video image as the progressive signals having 60 images every second into video signals in an odd field and an even field, interlaces the odd and even fields, and converts the images in the fields into the interlace video signal having 30 images every second (1/30 sec).

The P/I selector 15 selects and outputs any of the progressive video signal outputted from the frame memory 13 and the interlace video signal format-converted and generated by the interlace converting circuit 14 in accordance with the format of the monitor 5 connected to the output terminal of the P/I selector 15, and the monitor 5 regenerates and displays the moving image of the subject image picked up by the CCD 10.

The recording instructing switch 20 arranged to the camera head 3 is an operating switch which issues, to the frame memory 13, an instruction for stopping the updating of the temporary storage of the progressive video signal every frame (1/60 sec) and an instruction for driving the recording processing circuit 21.

The recording instructing switch 20 is turned on and then the frame memory 13 stops the operation for updating the storage every 1/60 sec, and holds the storage contents of the progressive video signal in the frame at the stop timing for a predetermined period (hereinafter, referred to as freezingly held). The progressive video signal in the frame freezingly held in the frame memory 13 is outputted to the interlace converting circuit 14 and the P/I selector 15, and is displayed on the monitor 5 connected to the P/I selector 15 as the still video image.

The recording processing circuit 21 is turned on from the recording instructing switch 20, then, converts the progressive video signal of the frame freezingly held in the frame memory 13 into data in a file format for the still image such as a JPEG or TIFF image in accordance with the recording instructing signal from the recording instructing switch 20, and records the converted data in the memory card 22 as a recording medium for the still image.

The memory card 22 is a medium formed by including a magnetic memory and a semiconductor memory, is detachable to the CCU 4, is attached to the personal computer 8 or the like, reads the data in the file format for the still image recorded in the memory card 22, and can regenerate and display the still image on a display device of the personal computer 8.

That is, when the affected part of the body cavity is picked up by the electronic endoscope and a desired picked-up image is recorded as the still image during the diagnosis and cure using the picked-up image, the operator turns on the recording instructing switch 20 arranged to the camera head 3. Then, at this timing, the progressive video signal in the frame stored in the frame memory 13 is freezingly held. The recording instructing switch 20 stops the operation for updating the temporary storage of the progressive video signal every frame (1/60 sec) and therefore is a switch for outputting a signal for instructing the generation of the still image. The progressive video signal of the frame freezingly held is converted into the data for the file format for the still image by the recording processing circuit 21 and is recorded to the memory card 22. Simultaneously, the P/I selector 15 selects any of the progressive video signal freezingly held in the frame memory 13 and the interlace video signal that is generated by format-converting the progressive video signal freezingly held by the interlace converting circuit 14 and thus the still image is displayed on the monitor 5. Consequently, the operator can visually confirm the still image instructed by the recording instructing switch 20.

With respect to the operation for freezingly holding the progressive video signal of one frame in the frame memory 13 and the operation for converting the progressive video signal of the frame freezingly held in the frame memory 13 into the data in the file format for the still image by the recording processing circuit 21, which are executed upon turning on the recording instructing switch 20, the recording instructing switch 20 is turned on, thereafter, a timer is operated, the time counting starts, it is determined whether or not the preset desired time passes, if the preset desired time passes, and then the operation for freezingly holding the signal and the operation for converting the signal into the data into the file format for the still image may automatically be stopped. Alternatively, the recording instructing switch 20 is turned on again and then the freezing operation of the frame memory 13 and the operation for converting the signal into the data in the file format for the still image by the recording processing circuit 21 may be stopped.

As mentioned above, the data for the still image recorded in the memory card 22 is read and reproduced by the personal computer 8, then, as shown in Fig. 2, a subject image 33 is displayed in the center of a monitor screen 30. Further, the monitor screen 30 displays the character information superimposed by the character information superimposing circuit 12, such as the name of the patient on the upper left thereof and date and the time 32 of the endoscope diagnosis on the upper right thereof. Therefore, the above display operation enables the file for the still image to easily be managed.

With the electronic endoscope apparatus with the above-mentioned structure, the still image for the diagnosis and cure using the endoscope is recorded on the progressive system with high image quality based on the progressive video signal picked up and generated on the progressive system which enables the capturing of the high-quality image. Further, the procedure situation of the diagnosis and cure using the endoscope can be displayed on any of the progressive-system monitor and the interlace-system one. That is, the frame memory 13 and the recording processing circuit 21 are controlled based on the signal from the recording instructing switch 20 as the operating switch, the still image outputted from the frame memory 13 is recorded to a predetermined medium, the moving image is switched to the still image, and the still image is outputted from the frame memory 13.

Incidentally, with the above-described structure, the P/I selector 15 selects and outputs the video signal on the scanning system corresponding to the monitor 5. However, a circuit for outputting the two signals of the progressive video signal and the interlace video signal may be arranged without using the P/I selector 15. That is, as the output circuit for outputting the two signals, an output terminal of the progressive video signal may be arranged on the output side of the frame memory 13, a terminal of the interlace video signal may be arranged on the output side of the interlace converting circuit 14, a progressive-system monitor may be connected to the terminal of the progressive video signal, and an interlace-system monitor may be connected to the terminal of the interlace video signal.

In the description of the electronic endoscope apparatus according to the first embodiment, the camera head 3 is detachable to the eye piece portion of the optical endoscope 2 as an example. Further, an electronic endoscope including the CCD at the edge portion of an inserting portion thereof may be used.

Next, a description is given of a modification of the first embodiment.

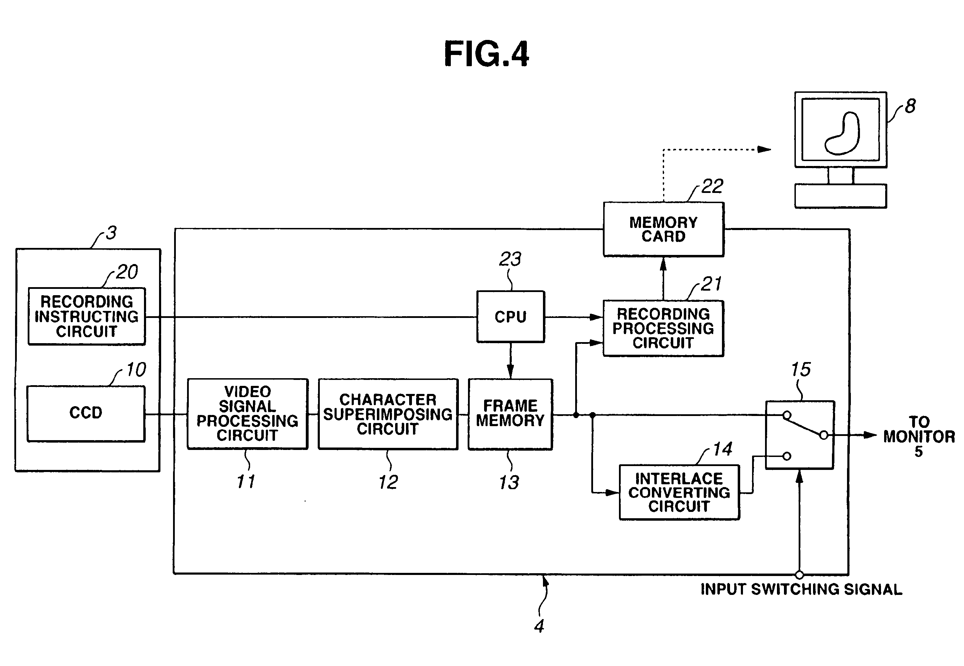

Fig. 4 is a block diagram showing the structure of an electronic endoscope apparatus according to the modification of the first embodiment of the present invention. Unlike the structure shown in Fig. 1, a central processing unit (hereinafter, referred to as a CPU) is arranged to receive the recording instructing signal from the recording instructing switch 20 and to output a control signal to the frame memory 13 and the recording processing circuit 21 based on the received recording instructing signal.

A CPU 23 receives the recording instructing signal from the recording instructing switch 20, then executes a program stored in a memory (not shown), synchronously with an image signal, stops the writing of the image data to the frame memory 13, and supplies, to the frame memory 13, a control signal for instructing the freezing so as to freezingly hold the image for a predetermined period.

Simultaneously, the CPU 23 supplies the control signal for instructing the recording to the recording processing circuit 21 based on the recording instructing signal from the recording instructing switch 20 so as to record the freezing image that is temporarily stored in the frame memory 13.

As described above, if the CPU 23 controls the frame memory 13 and also controls the recording processing circuit 21 based on the recording instructing signal from the recording instructing switch 20, the still image of the diagnosis and cure using the endoscope is recorded on the progressive system with high image quality based on the progressive video signal picked up and generated on the progressive system which enables the capturing the high-quality image. Further, the procedure situation of the diagnosis and cure using the endoscope can be displayed on any of the progressive-system monitor and the interlace-system monitor.

### (Second embodiment)

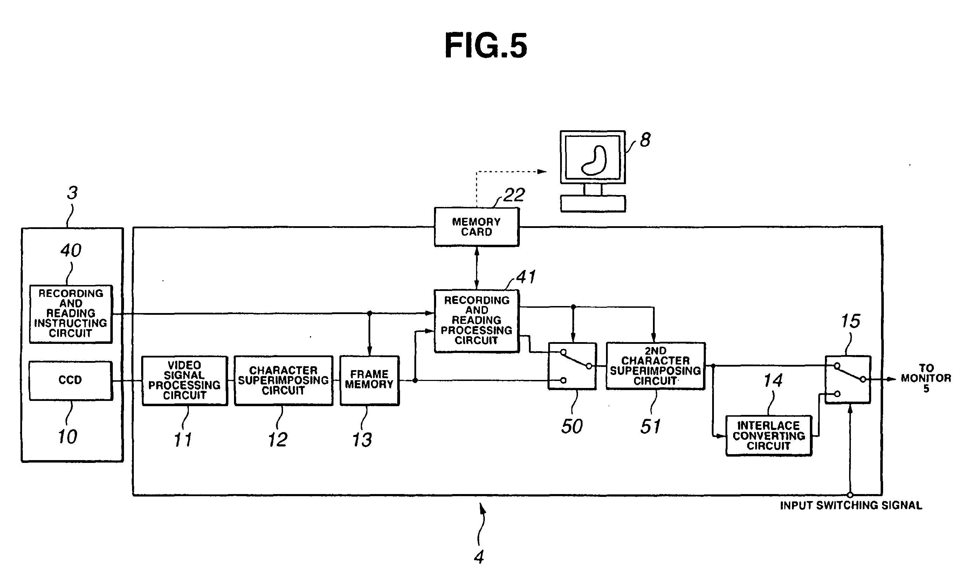

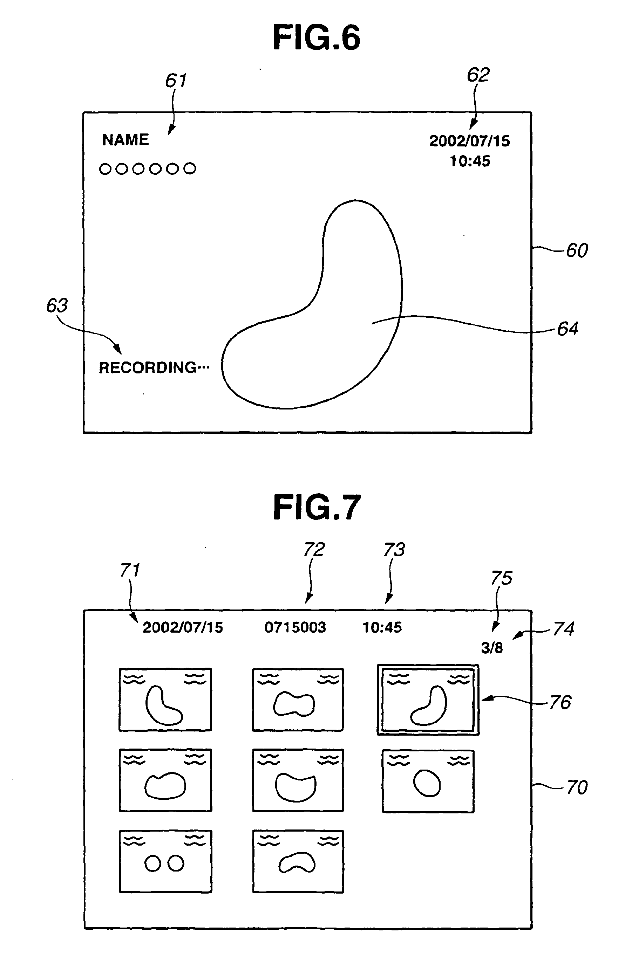

Next, an electronic endoscope apparatus will be described according to the second embodiment of the present invention with reference to Figs. 5 to 8. Fig. 5 is a block diagram showing the structure of the electronic endoscope apparatus according to the second embodiment of the present invention. Fig. 6 is an explanatory diagram describing a display screen of a subject still-image which is picked up by the electronic endoscope apparatus according to the second embodiment of the present invention. Fig. 7 is an explanatory diagram describing a display screen of a plurality of still images picked up by the electronic endoscope apparatus according to the second embodiment of the present invention. Incidentally, the same portions as those shown in Figs. 1 to 3 are designated by the same reference numerals and a detailed description thereof is omitted.

The camera head 3 in the electronic endoscope apparatus according to the second embodiment has the CCD 10 and a recording and regenerating instructing switch 40. The recording and regenerating instructing switch 40 includes a recording instructing switch for instructing the recording the still image and a regenerating instructing switch for instructing the regenerating of the video image which is recorded as the still image.

The progressive image pick-up signal outputted from the CCD 10 every 1/60 sec, namely, every frame is subjected to conversion processing into a predetermined progressive video signal by the video signal processing circuit 11. Then, the character superimposing circuit 12 superimposes the character information to the progressive video signal, and the superimposed signal is outputted to the frame memory 13. The progressive video signal having the superimposed character information is temporarily stored in the frame memory 13 based on the frame unit and the temporarily-stored progressive video signal is outputted if necessary. That is, the progressive video signals corresponding to the one frame are stored and outputted every 1/60 sec.

An output of the frame memory 13 is connected to the recording and regenerating processing circuit 41 and one input terminal of a moving/still images selector 50. An output of the recording and regenerating processing circuit 41 is connected to another input terminal of the moving /still images selector 50.

The frame memory 13 receives an instructing signal for recording the still image by turning on the recording instructing switch of the recording and regenerating instructing switch 40, and then freezingly holds the progressive video signal of the frame at the timing for receiving the instructing signal.

The recording and regenerating processing circuit 41 receives the instructing signal for recording the still image by operating the recording instructing switch of the recording and regenerating instructing switch 40, converts the progressive video signal of the frame freezingly held in the frame memory 13 into data in the file format for the still image, and records the converted data in the memory card 22.

Further, the recording and regenerating processing circuit 41 receives the instructing signal for reading the still image by turning on the regenerating instructing switch of the recording and regenerating instructing switch 40, then, reads and demodulates the file data for the still image recorded in the memory card 22, generates the progressive video signal for the still image, and outputs the generated signal to the moving/still images selector 50. Further, the recording and regenerating processing circuit 41 controls the moving/still images selector 50 so as to output the progressive video signal for the still image from the moving/still images selector 50, and also controls a second character superimposing circuit 51 so as to superimpose predetermined second character information to the progressive video signal for the still image outputted from the moving/still images selector 50.

The output of the second character superimposing circuit 51 is connected to one input terminal of the P/I selector 15 and to another input terminal via the interlace converting circuit 14.

The electronic endoscope apparatus having the above structure converts the progressive image pick-up signal picked up by the CCD 10 on the sequential scanning system of the camera head 3 into the progressive video signal by performing predetermined signal processing in the video signal processing circuit 11, thereby generating the progressive video signal.

The character superimposing circuit 12 superimposes the character information on screen menu such as a patient name and the current date and time to the progressive video signal. The progressive video signal having the superimposed character information is recorded and updated to the frame memory 13 every 1/60 sec, namely, every frame.

The progressive video signal every frame which is temporarily stored and outputted to the frame memory 13 is outputted to the recording and regenerating processing circuit 41 and one input terminal of the moving/still images selector 50.

When the recording and regenerating instructing switch 40 in the camera head 3 dose not input the instruction, the recording and regenerating processing circuit 41 controls the moving/still images selector 50 so as to select and output the progressive video signal which is inputted from the frame memory 13. Alternatively, the recording and regenerating processing circuit 41 stops the operation for superimposing the character information in the second character superimposing circuit 51, and the moving/still images selector 50 outputs the progressive video signal to the P/I selector 15 and the interlace converting circuit 14.

That is, similarly to the electronic endoscope apparatus according to the first embodiment, the P/I selector 15 outputs any of the progressive video signal that is generated by sequentially scanning and picking up by the CCD 10 and the interlace video signal that is obtained by format-converting the progressive video signal by the interlace converting circuit 14. The monitor 5 matching the format displays the moving image and also displays the patient name and the photographing date of the displayed moving image.

Next, an instruction for recording the still image is inputted by turning on the recording instructing switch of the recording and regenerating instructing switch 40 in the camera head 3. Then, the frame memory 13 temporarily stops storing the progressive video signal every frame and operating the output thereof and enters a freezing state in which the progressive video signal of the frame stored at the timing is held for a certain period.

The instructing signal for recording the still image is inputted from the recording and regenerating instructing switch 40 and then the recording and regenerating processing circuit 41 converts the progressive video signal of the frame freezingly held in the frame memory 13 into the data in the file format for the still image such as a JPEG or TIFF image, and records the converted data to the memory card 22 as the recording medium. Further, the connection is controlled so that the output from the moving/still images selector 50 is outputted to the recording and regenerating processing circuit 41. The recording and regenerating processing circuit 41 outputs, to the second character superimposing circuit 51, the progressive video signal of the frame which is converted into the data in the file format for the still image thereby.

The second character superimposing circuit 51 superimposes, to the progressive video signal from the recording and regenerating processing circuit 41, the character information such as "RECORDING..." for notifying the operator that the image is being currently recorded. Since the character information such as "RECORDING..." is character information which does not need to be recorded together with the still image, the second character information circuit 51 at the latter stage of the frame memory 13 superimposes the character information such as "RECORDING...".

The P/I selector 15 selects any of the progressive video signal for the still image having the superimposed second character information in the second character superimposing circuit 51 and the interlace video signal for the still image which is obtained by format-converting the progressive video signal for the still image by using the interlace converting circuit 14, and outputs the selected signal to the monitor 5.

That is, the instructing signal for recording the still image is inputted from the recording and regenerating instructing switch 40 and then the frame memory 13 freezingly holds the progressive video signal of the frame at the timing for inputting the instructing signal for recording the still image for a predetermined time. The recording and regenerating processing circuit 41 converts the progressive video signal of the frame freezingly held into data in the file format for recording the still image in the memory card 22, and executes the recording to the memory card 22. Further, when recording and regenerating processing circuit 41 displays, as the still image on the monitor 5, the progressive video signal of the frame recorded to the data in the file format for the still image, it controls the second character superimposing circuit 51 so as to superimpose the second character information indicating the progressive video signal is being recorded to the memory card 22 as the still image. Thus, referring to Fig. 6, a display screen 60 of the monitor 5 displays the picked-up subject image 64, patient name of the subject image 61, the image pick-up year, month, date, and time of the time 62, and the "RECORDING..." 63 indicating the data is being recorded and the operator of the electronic endoscope apparatus can confirm the contents of the still image and that the still image is being recorded.

Next, an instruction for regenerating the still image is inputted by turning on the regenerating instructing switch of the recording and regenerating instructing switch 40 in the camera head 3. Then, the recording and regenerating processing circuit 41 reads the entire data in the file format for the still image which is recorded in the memory card 22, and demodulates and generates the progressive video signal for the still image to simultaneously display the entire read data for the still image on the same screen. The recording and regenerating processing circuit 41 outputs the demodulated and generated progressive video signal for the still image to the P/I selector 15 and the interlace converting circuit 14 via the moving/still images selector 50 and the second character superimposing circuit 51.

Referring to Fig. 7, when the memory card 22 records, e.g., data for eight still images of the subject, the entire eight sill images of the subject are displayed on a display screen 70 of the monitor 5 with predetermined size and position.

The operator operates an operating switch (not shown) or the like, thereby instructing a still image 76 of the subject which is surrounded by double frames in Fig. 7 among the eight still images of the subject that are displayed on the display screen 70. Then, the display screen 70 displays a recording date 71 of the data for the still image of the still image 76 of the subject, still-image data file name 72, recording time 73, total number 74 of data for the still image recorded to the memory card 22, and ordering number 75 of the still image 76 of the subject designated from the total number of data for the still image.

The recording and regenerating processing circuit 41 superimposes the data recorded to the memory card 22 to the progressive video signal for the still image in the second character superimposing circuit 51 so as to display the date 71, file number 72, time 73, total number 74 of data for the still image, and ordering number 75 which are displayed as well as the plurality of still images on the display screen 70.

In the electronic endoscope apparatus with the above-described structure, the video signal for recording the still image is recorded to the memory card 22 on the progressive system with high image quality, and the progressive video signal recorded as the still image is displayed on the monitor by using the electronic endoscope apparatus in place of using the personal computer. Further, in the electronic endoscope apparatus, the progressive video signal is picked up and regenerated with high accuracy and high image quality for both the moving image and the still image. Furthermore, in the electronic endoscope apparatus, the image on the interlace system other than the progressive one can be displayed on the monitor.

As mentioned above according to the first embodiment, with respect to the operation for freezingly holding the progressive video signal of one frame into the frame memory 13 and the operation for converting the progressive video signal of the frame freezingly held in the frame memory 13 into the data in the file format for the still image in the recording and regenerating processing circuit 41 which are executed upon turning on the recording instructing switch according to the second embodiment, the recording instructing switch is turned on, then, the timer operates to start the time counting, it is determined whether or not preset desired time passes, when the preset desired time passes, the freezingly holding operation and the operation for conversion into the data in the file format for the still image may automatically be stopped. Alternatively, an on-signal is inputted again from the recording instructing switch and, then, the freezing operation of the frame memory 13 and the operation for conversion into the data in the file format for the still image in the recording and regenerating processing circuit 41 may be stopped.

Further, according to the second embodiment, the P/I selector 15 selects and outputs the video signal on the scanning system corresponding to the monitor 5. However, in place of using the P/I selector 15, a circuit for outputting the two signals of the progressive video signal and the interlace video signal may be arranged. That is, as the output circuit for outputting the two signals, an output terminal of the progressive video signal may be arranged on the output side of the frame memory 13, a terminal of the interlace video signal may be arranged on the output side of the interlace converting circuit 14, a progressive-system monitor may be connected to the terminal for the progressive video signal, and an interlace-system monitor may be connected to the terminal of the interlace video signal.

Next, a description is given of the structure of an electronic endoscope apparatus according to a modification of the second embodiment.

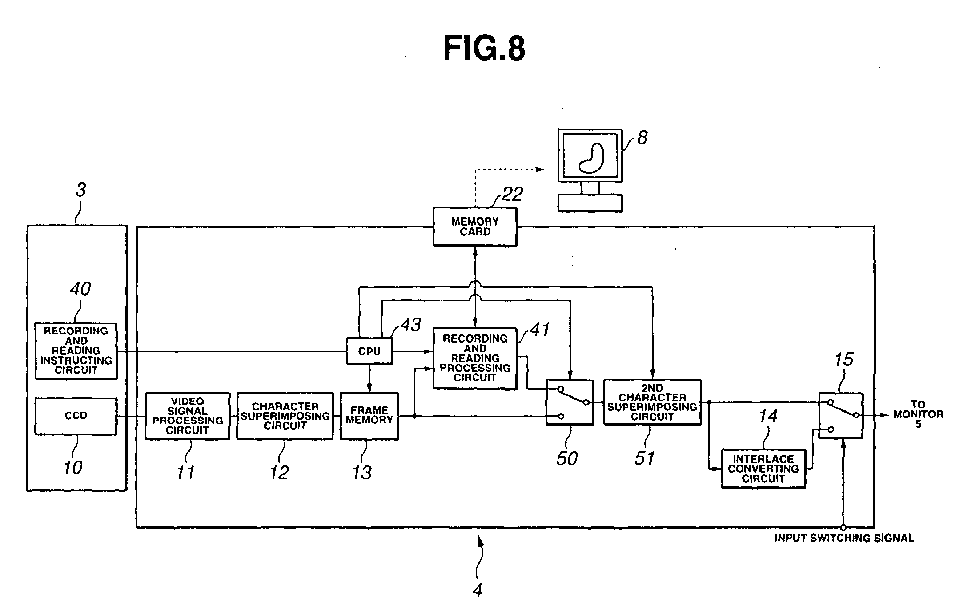

Fig. 8 is a block diagram showing the structure of the electronic endoscope apparatus according to the modification of the second embodiment of the present invention. Unlike the structure shown in Fig. 5, a CPU 43 is arranged to receive a recording instructing signal from the recording and regenerating instructing switch 40 and to output a control signal based on the received recording and regenerating instructing signal, to the frame memory 13, recording and regenerating circuit 41, a switch 50, and the second character superimposing circuit 51.

The recording instructing switch in the recording and regenerating instructing switch 40 is turned on, thereby receiving the instructing signal for recording the still image. Then, the CPU 43 stops the writing of the image data to the frame memory 13 synchronously with the image signal, and issues an freezing instruction so as to freeze the image for a predetermined period. The recording and regenerating processing circuit 41 starts to record the freezed image which is temporarily stored in the frame memory 13 based on the recording instructing signal from the CPU 43.

Further, the CPU 43 controls the moving/still images selector 50 so as to connect an input of the moving/still images selector 50 to an output of the recording and regenerating processing circuit 41. Simultaneously, the CPU 43 supplies the control signal so that the second character superimposing circuit 51 superimposes the character indicating the image is being recorded to the progressive video signal for the still image as the output of the moving/still images selector 50.

As a result, referring to Fig. 6, the patient name and a message indicating the image is being recorded are displayed on the monitor 5 as well as the still image.

A regenerating instructing switch of the recording and regenerating instructing switch 40 is turned on, thereby receiving the instructing signal for reading the still image. Then, the CPU 43 reads the entire data of the still images recorded in the memory card 22. Further, the CPU 43 controls the moving/still images selector 50 so as to connect an input of the moving/still images selector 50 to an output of the recording and regenerating processing circuit 41. The entire read data of the still images is supplied to the P/I selector 15 and the interlace converting circuit 14 via the moving/still images selector 50 and the second character superimposing circuit 51. Consequently, a screen shown in Fig. 7 is displayed on the monitor 5.

Further, the operator operates an operating switch (not shown), thereby designating the still image 76 of the subject surrounded by double frames in Fig. 7 among the eight still images of the subject displayed on the display screen 70 shown in Fig. 7. Then, the CPU 43 reads, from the memory card 22, information including the recording date 71 of the data for the still image of the still image 76 of the subject, still-image data file name 72, recording time 73, total number 74 of the data for the still image recorded in the memory card 22, ordering number 75 of the still image 76 of the subject designated among the total number of the data for the still image. Further, the CPU 43 controls the second character superimposing circuit 51 based on the read information and displays the information as shown in Fig. 7.

As mentioned above, if the CPU 43 controls the frame memory 13, the recording and regenerating processing circuit 41, moving/still images selector 50, and the second character superimposing circuit 51 based on the recording instructing signal and the regenerating instructing signal from the recording and regenerating instructing switch 40, the still image of the diagnosis and cure using the endoscope is recorded on the progressive system with high quality based on the progressive video signal picked up and generated on the progressive system on which the image with high quality is obtained. Further, the procedure situation of the diagnosis and cure using the endoscope can be displayed on any of the progressive-system monitor and the interlace-system monitor.

Advantageously, the electronic endoscope apparatus according to the present invention records, as the still image, the video signal picked up and generated on the progressive system with high image quality to obtain the still image with high image quality and excellent vertical resolution without blur, and can display the image on the monitor on the system except for the progressive one by the converting function to a system different from the progressive system.

The embodiments of the present invention have been described. However, the present invention is not limited to these and, obviously, can be changed without departing from the spirit of the present invention.

### Industrial Applicability

As mentioned above, besides endoscopes, the image processing apparatus according to the present invention can be applied to various apparatuses which display the picked-up image on the monitors different in the scanning systems for display and record the still image by the progressive video signal with the high image quality.

### Cross-reference to related applications

The present application is filed based on claiming priorities of (1) Japanese Patent Application No. 2002-268834 filed to Japan on 13th September, 2002, and (2) Japanese Patent Application No. 2003-200814 filed to Japan on 23rd July, 2003 based on the claiming priority of Japanese Patent Application No. 2002-268834 filed to Japan on 13th September on 2002. The disclosure contents of the above (1) and (2) are referred to the description, claims, and the drawings of the present application.

## Claims

1. An image processing apparatus comprising:
video signal processing means (11) which performs signal processing of an inputted image signal and generates a non-interlace video signal;
a memory (13) which can store one frame of the non-interlace video signals so as to generate a still image of the non-interlace video signal outputted from the video signal processing means (11);
recording processing means (21, 41) which records the still image outputted from the memory (13) to a predetermined medium (22);
a first operating switch (20, 40) which is operated to instruct the generation of the still image and outputs a signal for instructing the generation of the still image;
converting means (14) which converts, into an interlace video signal, the non-interlace video signal outputted from the memory (13) or from the recording processing means (21, 41) and outputs the converted signal; and
switching means (15) which selects between the interlace video signal outputted from the converting means and a non-interlace video signal outputted from the memory (13) or from the recording processing means (21, 41) and outputs a signal,
wherein the memory (13) and the recording processing means (21 41) are controlled based on a signal from the first operating switch (20, 40), the still image outputted from the memory (13) is recorded to the predetermined medium (22), the still image and a moving image are switched, and the switched image is outputted from the memory (13) or from the recording processing means (21, 41).

2. An image processing apparatus according to Claim 1, further comprising:
first character superimposing means (12),
wherein the first character superimposing means (12) superimposes first character information on the non-interlace video signal from the video signal processing means (11), and outputs the superimposed information to the memory (13).

3. An image processing apparatus according to Claim 1 or 2, wherein the switching of the still image and the moving image outputted from the memory (13) based on the signal from the first operating switch (20, 40) is performed so that the still image is outputted from the memory (13) for a predetermined time, and the moving image is outputted from the memory (13) when the predetermined time elapsed.

4. An image processing apparatus according to Claim 1 or 2, wherein the switching of the still image and the moving image outputted from the memory (13) based on the signal from the first operating switch (10, 40) is performed so that the signal is outputted from the first operating switch (20, 40), then, the still image is outputted from the memory (13), and further the signal is outputted from the first operating switch (20, 40), then, the moving image is outputted from the memory (13).

5. An image processing apparatus according to any one of Claims 1 to 4, wherein the switching means (15) selectively outputs the non-interlace video signal and the interlace video signal in accordance with a scanning system for displaying the video signal which can be regenerated and displayed on a monitor device (5) connected thereto.

6. An image processing apparatus according to any one of Claims 1 to 5, further comprising:
second character superimposing means (51),
wherein the second character superimposing means (51) superimposes second character information to the still image outputted from the recording processing means (41) and outputs the superimposed information to the converting means (14) and the switching means (15).

7. An image processing apparatus according to any one of Claims 1 to 6, further comprising:
a second operating switch (50),
wherein the second operating switch (50) is operated, then, the recording processing means (41) reads the still image recorded to the predetermined medium (22), and outputs the read image to the converting means (14) and switching means (15).

8. An image processing apparatus according to any one of Claims 1 to 7, wherein the inputted image signal is an image picked by an endoscope (2), and
the image processing apparatus is an image processing apparatus for an electronic endoscope apparatus (1).

9. An image pick-up apparatus comprising:
an image pick-up device (10) which picks up an image of a subject;
video signal processing means (11) which performs signal processing of an image signal of the image pick-up device (10) and generates a non-interlace video signal;
a memory (13) which can store one frame of the non-interlace video signals so as to generate a still image of the non-interlace video signal outputted from the video signal processing means (11);
recording processing means (21, 41) which records the still image outputted from the memory (13) to a predetermined medium (22);
a first operating switch (20, 40) which is operated to instruct the generation of the still image and outputs a signal for instructing the generation of the still image;
converting means (14) which converts, into an interlace video signal, a non-interlace video signal outputted from the memory (13) or from the recording processing means (21, 41), and outputs the converted signal; and
switching means (15) which selects between the interlace video signal outputted from the converting means (14) and the non-interlace video signal outputted from the memory (13) or from the recording processing means (21, 41) and outputs a signal,
wherein the memory (13) and the recording processing means (21, 41) are controlled based on a signal from the first operating switch (20, 40), the still image outputted from the memory (13) is recorded to the predetermined medium (22), the still image and a moving image are switched, and the switched image is outputted from the memory (13) or from the recording processing means (21, 41).

10. An image pick-up apparatus according to Claim 9, further comprising:
first character superimposing means (12),
wherein the first character superimposing means (12) superimposes first character information on the non-interlace video signal from the video processing means (11), and outputs the superimposed information to the memory (13).

11. An image pick-up apparatus according to Claim 9 or 10, wherein the switching of the still image and the moving image outputted from the memory (13) based on the signal from the first operating switch (20, 40) is performed so that the still image is outputted from the memory (13) for a predetermined time, and the moving image is outputted from the memory (13) when the predetermined time elapsed.

12. An image pick-up apparatus according to Claim 9 or 10, wherein the switching of the still image and the moving image outputted from the memory (13) based on the signal from the first operating switch (20, 40) is performed so that the signal is outputted from the first operating switch (20, 40), then, the still image is outputted from the memory (13), and further the signal is outputted from the first operating switch (20, 40), then, the moving image is outputted from the memory (13).

13. An image pick-up apparatus according to any one of Claims 9 to 12, wherein the switching means (15) selectively outputs the non-interlace video signal and the interlace video signal in accordance with a scanning system for displaying the video signal which can be regenerated and displayed on a monitor device (5) connected thereto.

14. An image pick-up apparatus according to any one of Claims 9 to 13, further comprising:
second character superimposing means (51),
wherein the second character superimposing means (51) superimposes second character information to the still image outputted from the recording processing means (41) and outputs the superimposed information to the converting means (14) and the switching means (15).

15. An image pick-up apparatus according to any one of Claims 9 to 14, further comprising:
a second operating switch (50),
wherein the second operating switch (50) is operated, then, the recording processing means (41) reads the still image recorded to the predetermined medium (22), and outputs the read image to the converting means (14) and switching means (15).

16. An image pick-up apparatus according to any one of Claims 9 to 15, wherein the inputted image signal is an image picked by an endoscope (2), and
the image processing apparatus is an image processing apparatus for an electronic endoscope apparatus (1).

## Patentansprüche

1. Bildverarbeitungsgerät, aufweisend:
ein Videosignalverarbeitungsmittel (11), das die Signalverarbeitung eines eingegebenen Bildsignals ausführt und ein fortlaufend abgetastetes Videosignal erzeugt;
einen Speicher (15), der ein Einzelbild der fortlaufend abgetasteten Videosignale speichern kann, um ein Standbild des fortlaufend abgetasteten Videosignals zu erzeugen, das vom Videosignalverarbeitungsmittel (11) ausgegeben wird;
ein Aufzeichnungsverarbeitungsmittel (21, 41), das das vom Speicher (13) ausgegebene Standbild auf einem vorgegebenen Medium (23) aufzeichnet;
einen ersten Operationsschalter (20, 40), der aktiviert wird, um die Erzeugung des Standbildes anzuweisen, und der ein Ausgangssignal zur Anweisung der Erzeugung des Standbildes ausgibt;
ein Wandlermittel (14), das das vom Speicher (13) oder vom Aufzeichnungsverarbeitungsmittel (21, 41) ausgegebene fortlaufend abgetastete Videosignal in ein Zeilensprung-Videosignal wandelt und das gewandelte Signal ausgibt; und
ein Schaltmittel (13), das zwischen dem vom Wandlermittel ausgegebenen Zeilensprung-Videosignal und einem vom Speicher (13) oder vom Aufzeichnungsverarbeitungsmittel (21, 41) ausgegebenen fortlaufend abgetasteten Videosignal wählt und ein Signal ausgibt,
bei dem der Speicher (13) und das Aufzeichnungsverarbeitungsmittel (21, 41) auf Basis eines Signals vom ersten Operationsschalter (20, 40) gesteuert werden, das vom Speicher (13) ausgegebene Standbild auf dem vorgegebenen Medium (22) aufgezeichnet wird, das Standbild und ein bewegtes Bild umgeschaltet werden, und
das umgeschaltete Bild aus dem Speicher (13) oder dem Aufzeichnungsverarbeitungsmittel (21, 41) ausgegeben wird.

2. Bildverarbeitungsgerät nach Anspruch 1, ferner aufweisend:
ein erstes Zeichenüberlagerungsmittel (12),
wobei das erste Zeichenüberlagerungsmittel (12) das fortlaufend abgetastete Videosignal vom Videosignalverarbeitungsmittel (11) mit ersten Zeicheninformationen überlagert und die überlagerten Informationen an den Speicher (13) ausgibt.

3. Bildverarbeitungsgerät nach Anspruch 1 oder 2, bei dem das Umschalten des Standbildes und des vom Speicher (13) auf Basis des Signals vom ersten Operationsschalter (20, 40) ausgegebenen bewegten Bildes so erfolgt, dass das Standbild vom Speicher (13) eine vorgegebene Zeit lang ausgegeben wird und das bewegte Bild vom Speicher (13) nach dem Ablauf der vorgegebenen Zeit ausgegeben wird.

4. Bildverarbeitungsgerät nach Anspruch 1 oder 2, bei dem das Umschalten des Standbildes und des vom Speicher (13) auf Basis des Signals vom ersten Operationsschalter (20, 40) ausgegebenen bewegten Bildes so erfolgt, dass das Signal vom ersten Operationsschalter (20, 40) ausgegeben wird, dann das Standbild vom Speicher (13) ausgegeben wird und weiter das Signal vom ersten Operationsschalter (20, 40) ausgegeben und dann das bewegte Bild vom Speicher (13) ausgegeben wird.

5. Bildverarbeitungsgerät nach einem der Ansprüche 1 bis 4, bei dem das Schaltmittel (15) das fortlaufend abgetastete Videosignal und das Zeilensprung-Videosignal gemäß einem Abtastsystem zur Anzeige des Videosignals selektiv ausgibt, das auf einem angeschlossenen Monitorgerät (5) regeneriert und angezeigt werden kann.

6. Bildverarbeitungsgerät nach einem der Ansprüche 1 bis 5, ferner aufweisend:
ein zweites Zeichenüberlagerungsmittel (51),
wobei das zweite Zeichenüberlagerungsmittel (51) das vom Aufzeichnungsverarbeitungsmittel (41) ausgegebene Standbild mit zweiten Zeicheninformationen überlagert und die überlagerten Informationen an das Wandlermittel (14) und das Schaltmittel (15) ausgibt.

7. Bildverarbeitungsgerät nach einem der Ansprüche 1 bis 6, ferner aufweisend:
einen zweiten Operationsschalter (50),
wobei dann, wenn der zweite Operationsschalter (50) aktiviert wird, das Aufzeichnungsverarbeitungsmittel (41) das auf dem vorgegebenen Medium (22) aufgezeichnete Bild liest und das gelesene Bild an das Wandlermittel (14) und das Schaltmittel (15) ausgibt.

8. Bildverarbeitungsgerät nach einem der Ansprüche 1 bis 7, bei dem das eingegebene Bildsignal ein von einem Endoskop (2) aufgenommenes Bild ist, und das Bildverarbeitungsgerät ein Bildverarbeitungsgerät für ein elektronisches Endoskopgerät (1) ist.

9. Bildaufnahmegerät, aufweisend:
eine Bildaufnahmeeinrichtung (10), die ein Bild eines Objektes aufnimmt;
ein Videosignalverarbeitungsmittel (11), das die Signalverarbeitung eines Bildsignals der Bildaufnahmeeinrichtung (10) ausführt und ein fortlaufend abgetastetes Videosignal erzeugt;
einen Speicher (13), der ein Einzelbild der fortlaufend abgetasteten Videosignale speichern kann, um ein Standbild des fortlaufend abgetasteten Videosignals zu erzeugen, das vom Videosignalverarbeitungsmittel (11) ausgegeben wird;
ein Aufzeichnungsverarbeitungsmittel (21, 41), das das vom Speicher (13) ausgegebene Standbild auf einem vorgegebenen Medium (22) aufzeichnet;
einen ersten Operationsschalter (20, 40), der aktiviert wird, um die Erzeugung des Standbildes anzuweisen, und der ein Ausgangssignal zur Anweisung der Erzeugung des Standbildes ausgibt;
ein Wandlermittel (14), das das vom Speicher (13) oder vom Aufzeichnungsverarbeitungsmittel (21, 41) ausgegebene fortlaufend abgetastete Videosignal in ein Zeilensprung-Videosignal wandelt und das gewandelte Signal ausgibt; und
ein Schaltmittel (15), das zwischen dem vom Wandlermittel (14) ausgegebenen Zeilensprung-Videosignal und dem vom Speicher (13) oder vom Aufzeichnungsverarbeitungsmittel (21, 41) ausgegebenen fortlaufend abgetasteten Videosignal wählt und ein Signal ausgibt,
bei dem der Speicher (13) und das Aufzeichnungsverarbeitungsmittel (21, 41) auf Basis eines Signals vom ersten Operationsschalter (20, 40) gesteuert werden, das vom Speicher (13) ausgegebene Standbild auf dem vorgegebenen Medium (22) aufgezeichnet wird, das Standbild und ein bewegtes Bild umgeschaltet werden, und
das umgeschaltete Bild aus dem Speicher (13) oder dem Aufzeichnungsverarbeitungsmittel (21, 41) ausgegeben wird.

10. Bildaufnahmegerät nach Anspruch 9, ferner aufweisend:
ein erstes Zeichenüberlagerungsmittel (12),
wobei das erste Zeichenüberlagerungsmittel (12) das fortlaufend abgetastete Videosignal vom Videosignalverarbeitungsmittel (11) mit ersten Zeicheninformationen überlagert und die überlagerten Informationen an den Speicher (13) ausgibt.

11. Bildaufnahmegerät nach Anspruch 9 oder 10, bei dem das Umschalten des Standbildes und des vom Speicher (13) auf Basis des Signals vom ersten Operationsschalter (20, 40) ausgegebenen bewegten Bildes so erfolgt, dass das Standbild vom Speicher (13) eine vorgegebene Zeit lang ausgegeben wird und das bewegte Bild vom Speicher (13) nach dem Ablauf der vorgegebenen Zeit ausgegeben wird.

12. Bildaufnahmegerät nach Anspruch 9 oder 10, bei dem das Umschalten des Standbildes und des vom Speicher (13) auf Basis des Signals vom ersten Operationsschalter (20, 40) ausgegebenen bewegten Bildes so erfolgt, dass das Signal vom ersten Operationsschalter (20, 40) ausgegeben wird, dann das Standbild vom Speicher (13) ausgegeben wird und weiter das Signal vom ersten Operationsschalter (20, 40) ausgegeben und dann das bewegte Bild vom Speicher (13) ausgegeben wird.

13. Bildaufnahmegerät nach einem der Ansprüche 9 bis 12, bei dem das Schaltmittel (15) das fortlaufend abgetastete Videosignal und das Zeilensprung-Videosignal gemäß einem Abtastsystem zur Anzeige des Videosignals selektiv ausgibt, das auf einem angeschlossenen Monitorgerät (5) regeneriert und angezeigt werden kann.

14. Bildaufnahmegerät nach einem der Ansprüche 9 bis 13, ferner aufweisend:
ein zweites Zeichenüberlagerungsmittel (51),
wobei das zweite Zeichenüberlagerungsmittel (51) das vom Aufzeichnungsverarbeitungsmittel (41) ausgegebene Standbild mit zweiten Zeicheninformationen überlagert und die überlagerten Informationen an das Wandlermittel (14) und das Schaltmittel (15) ausgibt.

15. Bildaufnahmegerät nach einem der Ansprüche 9 bis 14, ferner aufweisend:
einen zweiten Operationsschalter (50),
wobei dann, wenn der zweite Operationsschalter (50) aktiviert wird, das Aufzeichnungsverarbeitungsmittel (41) das auf dem vorgegebenen Medium (22) aufgezeichnete Bild liest und das gelesene Bild an das Wandlermittel (14) und das Schaltmittel (15) ausgibt.

16. Bildaufnahmegerät nach einem der Ansprüche 9 bis 15, bei dem das eingegebene Bildsignal ein von einem Endoskop (2) aufgenommenes Bild ist, und das Bildverarbeitungsgerät ein Bildverarbeitungsgerät für ein elektronisches Endoskopgerät (1) ist.

## Revendications

1. Appareil de traitement d'images comprenant :
un moyen de traitement de signaux vidéo (11) qui effectue un traitement de signal d'un signal d'image entré et génère un signal vidéo non entrelacé ;
une mémoire (13) qui peut stocker une trame des signaux vidéo non entrelacés de manière à générer une image fixe du signal vidéo non entrelacé délivré en sortie du moyen de traitement de signaux vidéo (11) ;
un moyen de traitement d'enregistrement (21, 41) qui enregistre l'image fixe délivrée en sortie de la mémoire (13) sur un support prédéterminé (22) ;
un premier commutateur de commande (20, 40) qui est utilisé pour ordonner la génération de l'image fixe et délivre en sortie un signal pour ordonner la génération de l'image fixe ;
un moyen de conversion (14) qui convertit, en un signal vidéo entrelacé, le signal vidéo non entrelacé délivré en sortie de la mémoire (13) ou du moyen de traitement d'enregistrement (21, 41) et délivre en sortie le signal converti ; et
un moyen de commutation (15) qui effectue une sélection entre le signal vidéo entrelacé délivré en sortie du moyen de conversion et un signal vidéo non entrelacé délivré en sortie de la mémoire (13) ou du moyen de traitement d'enregistrement (21, 41) et délivre en sortie un signal,
dans lequel la mémoire (13) et le moyen de traitement d'enregistrement (21, 41) sont commandés sur la base d'un signal provenant du premier commutateur de commande (20, 40), l'image fixe délivrée en sortie de la mémoire (13) est enregistrée sur le support prédéterminé (22), l'image fixe et une image animée sont commutées, et l'image commutée est délivrée en sortie de la mémoire (13) ou du moyen de traitement d'enregistrement (21, 41).

2. Appareil de traitement d'images selon la revendication 1, comprenant en outre :
un moyen de superposition de premier caractère (12),
dans lequel le moyen de superposition de premier caractère (12) superpose des informations de premier caractère sur le signal vidéo non entrelacé provenant du moyen de traitement de signaux vidéo (11), et délivre en sortie les informations superposées dans la mémoire (13).

3. Appareil de traitement d'images selon la revendication 1 ou 2, dans lequel la commutation de l'image fixe et de l'image animée délivrées en sortie de la mémoire (13) sur la base du signal provenant du premier commutateur de commande (20, 40) est effectuée de manière à ce que l'image fixe soit délivrée en sortie de la mémoire (13) pendant une durée prédéterminée, et l'image animée est délivrée en sortie de la mémoire (13) lorsque la durée prédéterminée est écoulée.

4. Appareil de traitement d'images selon la revendication 1 ou 2, dans lequel la commutation de l'image fixe et de l'image animée délivrées en sortie de la mémoire (13) sur la base du signal provenant du premier commutateur de commande (20, 40) est effectuée de manière à ce que le signal soit délivré en sortie du premier commutateur de commande (20, 40), ensuite l'image fixe est délivrée en sortie de la mémoire (13), et en outre le signal soit délivré en sortie du premier commutateur de commande (20, 40), ensuite l'image animée est délivrée en sortie de la mémoire (13).

5. Appareil de traitement d'images selon l'une quelconque des revendications 1 à 4, dans lequel le moyen de commutation (15) délivre sélectivement en sortie le signal vidéo non entrelacé et le signal vidéo entrelacé en fonction d'un système de balayage pour afficher le signal vidéo qui peut être régénéré et affiché sur un dispositif de moniteur (5) connecté à celui-ci.

6. Appareil de traitement d'images selon l'une quelconque des revendications 1 à 5, comprenant en outre :
un moyen de superposition de deuxième caractère (51),
dans lequel le moyen de superposition de deuxième caractère (51) superpose des informations de deuxième caractère sur l'image fixe délivrée en sortie du moyen de traitement d'enregistrement (41), et délivre en sortie les informations superposées dans le moyen de conversion (14) et le moyen de commutation (15).

7. Appareil de traitement d'images selon l'une quelconque des revendications 1 à 6, comprenant en outre :
un deuxième commutateur de commande (50),
dans lequel le deuxième commutateur de commande (50) est utilisé, ensuite le moyen de traitement d'enregistrement (41) lit l'image fixe enregistrée sur le support prédéterminé (22) et délivre en sortie l'image lue dans le moyen de conversion (14) et le moyen de commutation (15).

8. Appareil de traitement d'images selon l'une quelconque des revendications 1 à 7, dans lequel le signal d'image entré est une image prise par un endoscope (2), et
l'appareil de traitement d'images est un appareil de traitement d'images pour un endoscope électronique (1).

9. Appareil de prise d'images comprenant :
un dispositif de prise d'images (10) qui prend une image d'un sujet ;
un moyen de traitement de signaux vidéo (11) qui effectue un traitement de signal d'un signal d'image du dispositif de prise d'images (10) et génère un signal vidéo non entrelacé ;
une mémoire (13) qui peut stocker une trame des signaux vidéo non entrelacés de manière à générer une image fixe du signal vidéo non entrelacé délivré en sortie du moyen de traitement de signaux vidéo (11) ;
un moyen de traitement d'enregistrement (21, 41) qui enregistre l'image fixe délivrée en sortie de la mémoire (13) sur un support prédéterminé (22) ;
un premier commutateur de commande (20, 40) qui est utilisé pour ordonner la génération de l'image fixe et délivre en sortie un signal pour ordonner la génération de l'image fixe ;
un moyen de conversion (14) qui convertit, en un signal vidéo entrelacé, un signal vidéo non entrelacé délivré en sortie de la mémoire (13) ou du moyen de traitement d'enregistrement (21, 41) et délivre en sortie le signal converti ; et
un moyen de commutation (15) qui effectue une sélection entre le signal vidéo entrelacé délivré en sortie du moyen de conversion (14) et le signal vidéo non entrelacé délivré en sortie de la mémoire (13) ou du moyen de traitement d'enregistrement (21, 41) et délivre en sortie un signal,
dans lequel la mémoire (13) et le moyen de traitement d'enregistrement (21, 41) sont commandés sur la base d'un signal provenant du premier commutateur de commande (20, 40), l'image fixe délivrée en sortie de la mémoire (13) est enregistrée sur le support prédéterminé (22), l'image fixe et une image animée sont commutées, et l'image commutée est délivrée en sortie de la mémoire (13) ou du moyen de traitement d'enregistrement (21, 41).

10. Appareil de prise d'images selon la revendication 9, comprenant en outre :
un moyen de superposition de premier caractère (12),
dans lequel le moyen de superposition de premier caractère (12) superpose des informations de premier caractère sur le signal vidéo non entrelacé provenant du moyen de traitement de vidéo (11), et délivre en sortie les informations superposées dans la mémoire (13).

11. Appareil de prise d'images selon la revendication 9 ou 10, dans lequel la commutation de l'image fixe et de l'image animée délivrées en sortie de la mémoire (13) sur la base du signal provenant du premier commutateur de commande (20, 40) est effectuée de manière à ce que l'image fixe soit délivrée en sortie de la mémoire (13) pendant une durée prédéterminée, et l'image animée est délivrée en sortie de la mémoire (13) lorsque la durée prédéterminée est écoulée.

12. Appareil de prise d'images selon la revendication 9 ou 10, dans lequel la commutation de l'image fixe et de l'image animée délivrées en sortie de la mémoire (13) sur la base du signal provenant du premier commutateur de commande (20, 40) est effectuée de manière à ce que le signal soit délivré en sortie du premier commutateur de commande (20, 40), ensuite l'image fixe est délivrée en sortie de la mémoire (13), et en outre le signal soit délivré en sortie du premier commutateur de commande (20, 40), ensuite l'image animée est délivrée en sortie de la mémoire (13).

13. Appareil de prise d'images selon l'une quelconque des revendications 9 à 12, dans lequel le moyen de commutation (15) délivre sélectivement en sortie le signal vidéo non entrelacé et le signal vidéo entrelacé en fonction d'un système de balayage pour afficher le signal vidéo qui peut être régénéré et affiché sur un dispositif de moniteur (5) connecté à celui-ci.

14. Appareil de prise d'images selon l'une quelconque des revendications 9 à 13, comprenant en outre :
un moyen de superposition de deuxième caractère (51),
dans lequel le moyen de superposition de deuxième caractère (51) superpose des informations de deuxième caractère sur l'image fixe délivrée en sortie du moyen de traitement d'enregistrement (41) et délivre en sortie les informations superposées dans le moyen de conversion (14) et le moyen de commutation (15).

15. Appareil de prise d'images selon l'une quelconque des revendications 9 à 14, comprenant en outre :
un deuxième commutateur de commande (50),
dans lequel le deuxième commutateur de commande (50) est utilisé, ensuite le moyen de traitement d'enregistrement (41) lit l'image fixe enregistrée sur le support prédéterminé (22), et délivre en sortie l'image lue dans le moyen de conversion (14) et le moyen de commutation (15).

16. Appareil de prise d'images selon l'une quelconque des revendications 9 à 15, dans lequel le signal d'image entré est une image prise par un endoscope (2), et
l'appareil de traitement d'images est un appareil de traitement d'images pour un endoscope électronique (1).
